# EUROPEAN PATENT APPLICATION

(11) **EP 3 799 886 A1**
(43) Date of publication of application: **07.04.2021**
(21) Application number: 19784246.1
(22) Date of filing: 12.04.2019
(51) Int. Cl.: A61K 47/50, A61K 38/00, C08L 25/06, B82Y 5/00

(54) **MULTIFUNCTIONAL NANOPARTICLES FOR THERAGNOSIS**

(30) Priority: 12.04.2018 ES 201830360; 15.10.2018 ES 201830993
(71) Applicant: Universidad de Granada, 18071 Granada (ES)
(72) Inventor: SÁNCHEZ MARTÍN, Rosario María, 18071 Granada (ES); MARCHAL CORRALES, Juan Antonio, 18071 Granada (ES); DÍAZ MOCHÓN, Juan José, 18071 Granada (ES); CANO CORTÉS, Victoria, 18071 Granada (ES); NAVARRO MARCHAL, Saúl Abenhamar, 18071 Granada (ES); RUIZ BLAS, Maria Paz, 18071 Granada (ES)
(74) Representative: Hoffmann Eitle
(86) International application number: PCT/ES2019/070259
(87) International publication number: WO 2019/197702

(57) **Abstract**

The present invention relates to the field of medicine, particularly to functionalised nanoparticles (NP) for use in cancer therapy (treatment or diagnosis), to pharmaceutical compositions or nano devices comprising same, and also to a method for obtaining said functionalised nanoparticles. The nanoparticles described in the present invention can be used specifically to treat cancer efficiently, as same can selectively detect tumour cells.

## Description

### FIELD OF THE ART

The present invention is comprised in the medical area and relates to functionalised nanoparticles (NPs) and use thereof in theragnosis (treatment and diagnosis) of cancer, to pharmaceutical formulations or nanodevices comprising same, and also to methods for obtaining said functionalised nanoparticles. The nanoparticles described in the present invention can be used specifically to treat cancer efficiently, as they can selectively detect tumour cells.

### STATE OF THE ART

### Active tumour targeting

The therapeutic efficacy of tumour-targeting drug release systems is not optimised. The first reason is the poor cellular uptake in the tumour. The display of a specific tumour ligand, such as antibodies (Ab), Ab fragments, peptides, aptamers, polysaccharides, saccharides, folic acid, and other compounds, on the surface of NPs may increase NP retention and accumulation in the tumour vascular system, giving rise to an efficient and selective uptake into tumour cells, a process known as *"active tumour targeting". In vivo* and *in vitro* studies have demonstrated that NPs actively targeted by means of a ligand with different drug formulations exhibit an increase, though in varying degrees, in their therapeutic action when compared with the passive form. In addition to tumour cell targeting, tumour neovasculature represents another interesting target for cancer chemotherapy, such as tumour angiogenesis which is known to be extremely important in solid tumour growth and metastasis. The obliteration of tumour neovasculature will result in the shrinkage of the solid tumour established by blocking the blood supply (selective deprivation of cancer). In recent years, different types of targeted ligands such as cyclic RGD (cRGD), folic acid (FA), hyaluronic acid (HA), human epidermal receptor 2 (Her2), galactose glycyrrhizin, bisphosphonates, and (S,S-2-(3-(5-amino-1-carboxypentyl)ureido)-pentanedioic acid (ACUPA) have been used for releasing drugs actively targeting the tumour.

### Release targeting tumour vasculature

Tumour vasculature, which is structurally and functionally different compared with normal tissue vasculature, is highly disorganised with surprisingly convoluted and fenestrated blood vessels. In fact, vessels that form again are discontinuous, leaky, and exhibit an irregular expression of various molecules such as integrins, endothelial cell growth factor receptors, cell surface proteoglycans, proteases, and cellular matrix components. Specifically, proteins functionally important for tumour angiogenesis, which represent potential targets with respect to those targeted by anti-angiogenic therapy, are vascular endothelial growth factor receptors (VEGFR), integrins αvβ₃ and αvβ₅, Delta-like ligand 4 (DDL4), ephrin B4 (Eph-E34), ephrin B2 (Eph-B2), tumour endothelial markers 5 and 8 (TEM 5, TEM 8), annexin A1 (ANXA 1), and extra domain B-containing fibronectin (FN-ED-B).

The phage display technique has been widely used for analysing the structural and molecular diversity of normal and tumour vasculature. The purpose of a phage display study is to find the target peptides of the vasculature. To date, sequences capable of targeting the vasculature of normal tissues or organs, such as the brain, kidney, lung, muscles, pancreas, thymus, and mammary glands, have been found.

In particular, the RGD peptide targets tumour vasculature selectively expressing integrins αvβ₃ and αvβ₅. Integrins αvβ₃ and αvβ₅ are overexpressed in the vasculature associated with tumour and glioma cells and their activation triggers pathways responsible for tumour-induced angiogenesis and cell proliferation. The anti-angiogenic and anti-tumour effects of peptides containing the RGD domain, as well as their efficacy in imaging applications, have been tested in preclinical and clinical models.

### Multifunctional nanoparticles and theragnosis

Recent applications of multifunctional nanoparticles focus on the integration of diagnosis and therapy in a nanodevice, referred to as theragnosis. This approach allows performing diagnosis along with treatment and control of treatment efficacy and progression of the disease at the same time. Diagnostic applications in cancer therapy, such as chemotherapy, photodynamic therapy, siRNA therapy, and photothermal therapy have been published.

However, polymer nanoparticles for diagnosis known in the prior art (gold NPs, PLGA NPs, etc.) comprise both the drug and the fluorophore heterogeneously encapsulated inside the particle and PEGylation takes place by means of physical adsorption or by means of hydrogen bonds with respect to the nanoparticle. Lastly, the ligand for targeted release conjugates with the surface of the nanoparticle. This heterogeneous encapsulation limits system versatility since compatibility of the drug with the fluorophore must be taken into account, and moreover, as the drug is released into the tumour itself, the signal of the fluorophore is also weakened as they are encapsulated together. In contrast, the controlled multifunctionalisation of the nanoparticle allows adjusting the ratio between the amounts of each of the components: drug, ligand, and diagnostic agent. This considerably increases the possibilities of developing more effective nanodevices for controlled release and real-time monitoring.

### BRIEF DESCRIPTION OF THE INVENTION

The present invention relates to polymer nanoparticles (N) (hereinafter, nanoparticles of the invention) functionalised with (a) at least one imaging agent (T), and at least one bioactive molecule (D), and at least one ligand (L). Particularly, the present invention provides a diagnostic nanodevice comprising a nanoparticle containing a useful therapeutic load, an imaging agent, and a ligand for targeted release.

The nanoparticle may have an organic or inorganic composition. Organic nanoparticles can be nanoparticles based on polymers, include polystyrene (PS) functionalised with amino groups, polylactic acid (PLA), poly(lactic-co-glycolic acid) (PLGA), poly(N-vinylpyrrolidone) (PVP), polyethylene glycol (PEG), polycaprolactone, polyacrylic acid (PAA), poly(methyl methacrylate), and polyacrylamide. Additionally, natural polymers that can be used for preparing the nanoparticles include chitosan, gelatin, sodium alginate, and albumin. The use of polystyrene functionalised with amino groups is particularly suitable for the purposes of this invention and can be obtained, for example, by means of a dispersion polymerisation process (Sanchez-Martin et al., 2005, ChemBioChem, 6, 1341-1345).

Furthermore, the present invention describes a method for producing these functionalised polystyrene nanoparticles (NPs).

As an example to illustrate the invention, the nanoparticle of the invention can be used for treating breast cancer. In this case, the bioactive molecule (D) would be doxorubicin and the ligand (L) would be a breast cancer tissue-specific peptide sequence. The main advantage of the nanoparticle of the invention is its stability in an aqueous suspension with minimal particle agglomeration. This allows a multifunctionalisation of the nanoparticles to be carried out efficiently given that bioactive loads are generally stable in water. Furthermore, the nanoparticle of the invention offers the possibility of generically carrying out multifunctionalisation in organic solvents and using orthogonal strategies to conjugate the bioactive molecule (D) and control the amount of each of these bioactive molecules that is incorporated. Therefore, an important feature of the nanodevice of the invention is its versatility given that by simply changing or adapting the bioactive molecule (D), it can be adapted for diagnosis or for therapy and can also target different types of cancer depending on the ligand (L) ultimately chosen. Furthermore, the dosage regimen of the bioactive molecule (D), the selection of the ligand (L), and the imaging agent (T) can be modified and optimised with the nanotechnology used in the invention. This would allow the system to be readily scaled in a versatile and reproducible manner.

Accordingly, the nanoparticles of the invention are an effective and non-toxic tool for the treatment or diagnosis of cancer *in vivo.* In particular, they can be considered a selective anti-tumour therapy which is also complemented with the control of treatment efficacy (degree of tumour reduction) and drug release kinetics. Therefore, the nanoparticles of the invention will allow the integration in a single therapeutic agent of the following features: pharmacological selectivity, tissue specificity, and personalised treatment.

Furthermore, the present invention also relates to a nanodevice or a pharmaceutical composition comprising the nanoparticles of the invention, to the use thereof in the treatment, monitoring, and diagnosis of cancer.

### BRIEF DESCRIPTION OF THE FIGURES

Figure 1. Scheme showing the process for the functionalisation of aminomethylated polystyrene nanoparticles (NPs-1). The NPs-1 (Cy7-NPs) were functionalised with Cy7 (cyanine 7).
Figure 2. Scheme showing the process for the functionalisation of aminomethylated polystyrene nanoparticles (NPs-2). The NPs-2 (HP-Cy7-NPs) were functionalised with the homing peptide (HP) and Cy7.
Figure 3. Scheme showing the process for the functionalisation of aminomethylated polystyrene nanoparticles (NPs-3). The NPs-3 (Dox-Cy7-NPs) were functionalised with doxorubicin (Dox) and Cy7.
Figure 4. General scheme of the synthesis of the trifunctionalised nanoparticles of the invention. The NPs-4 (Dox-HP-Cy7-NPs) were functionalised with HP, Dox, and Cy7.
Figure 5. a) SEM analysis of the nanoparticles. b) AFM
Figure 6. Release of doxorubicin from Dox-HP-Cy7-NPs at pH 7.4 and pH 5.2 in 10% FBS. Error bars: mean ± S.D.

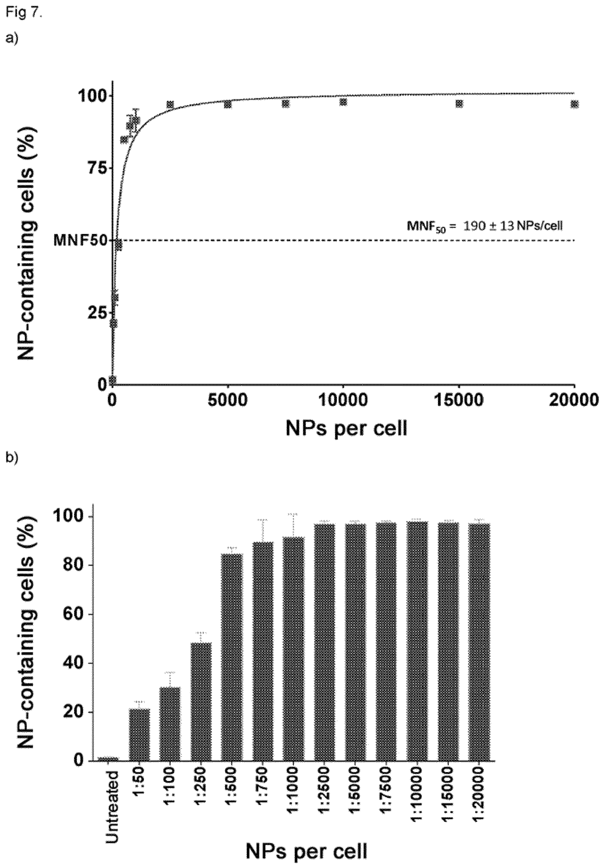

Figure 7. Efficiency of the cellular uptake of nanoparticles of the invention by MDA-MB-231 cells (24 hours of incubation).
Figure 8. Comparison between the efficacy of the uptake of the nanoparticle of the invention which is a trifunctionalised nanoparticle Dox-HP-Cy7-NP (NP-4) and bifunctionalised nanoparticles: Dox-Cy7-NPs (NPs-3), HP-Cy7-NPs (NPs-2), and monofunctionalised nanoparticles: Cy7-NPs (NPs-1).

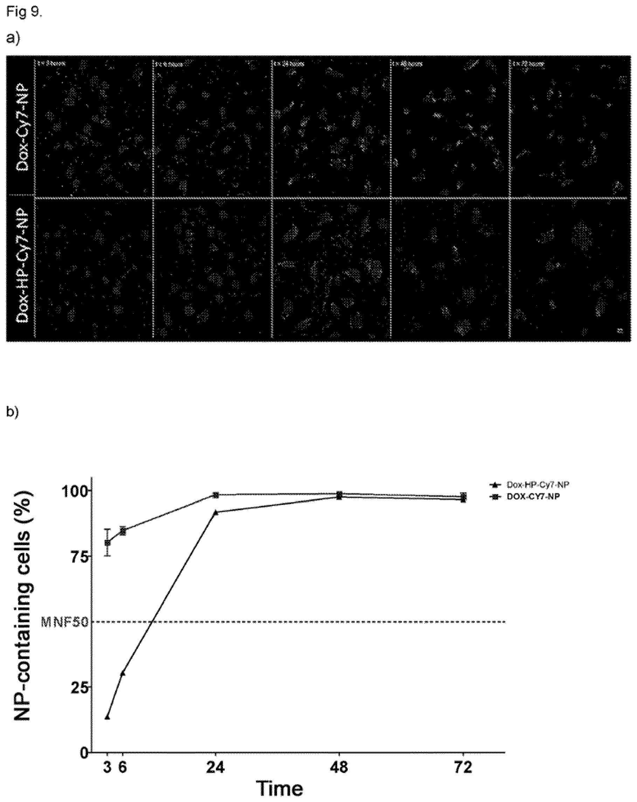

Figure 9. Analysis of incubation time versus uptake efficiency (% of NP-containing cells). (a) Confocal microscopy analysis (b) Flow cytometry analysis.
Figure 10. Analysis of the anti-proliferation efficacy of the nanoparticle of the invention compared with the drug in solution. Calculation of the IC₅₀ value.

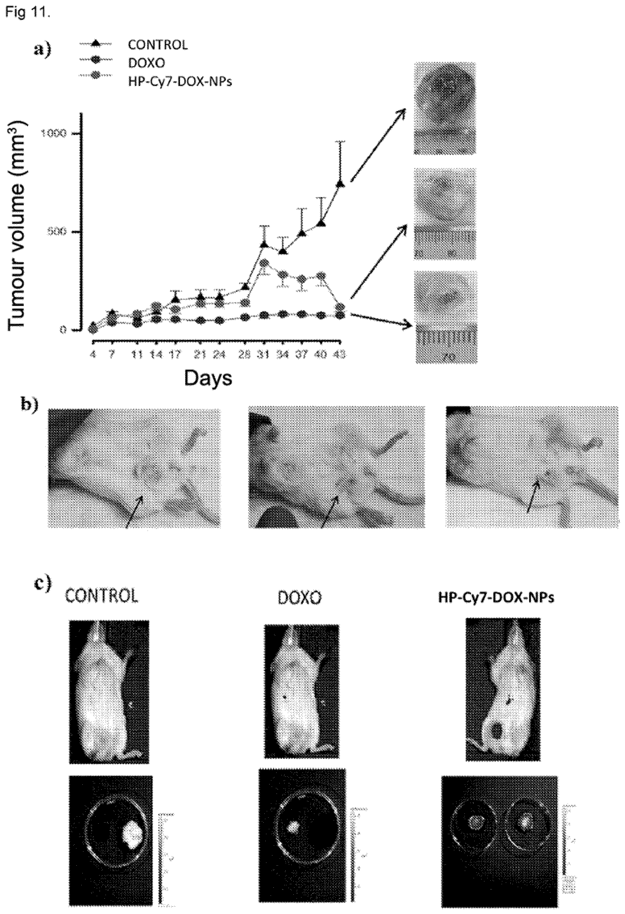

Figure 11. *In vivo* evaluation of the therapeutic efficacy of the intravenous administration of theranostic NPs (HP-Cy7-DOX-NPs (14)) compared with doxorubicin in solution and PBS in NSG mice with breast tumours induced by means of inoculating cells from the MDA-MB-231 line in matrigel. A) Depiction of the relative change of tumour volume (mm³) over time of each group of mice during 43 days of treatment. Each dot and vertical line represents mean ± SEM (n = 5 per group). Statistically significant differences between the groups treated with PBS and with free DOX *P≤0.05; **P≤0.01; and PBS versus HP-Cy7-DOX-NPs #P≤0.05; ##P≤0.01 on the same day after treatment (two-way repeated measures ANOVA followed by the Bonferroni test). The time when the tumour reached a size of 100 mm³ was considered time zero. Periodic administrations are performed intravenously to each group of mice every 3 days. B) Images of orthotopic tumours in the breast of untreated mice (top image) and mice treated with trifunctionalised NPs (bottom image) where a lower tumour volume is observed. C) *In vivo* fluorescence analysis (IVIS) for the detection of nanoparticles compared with negative controls in mice and isolated tumours.

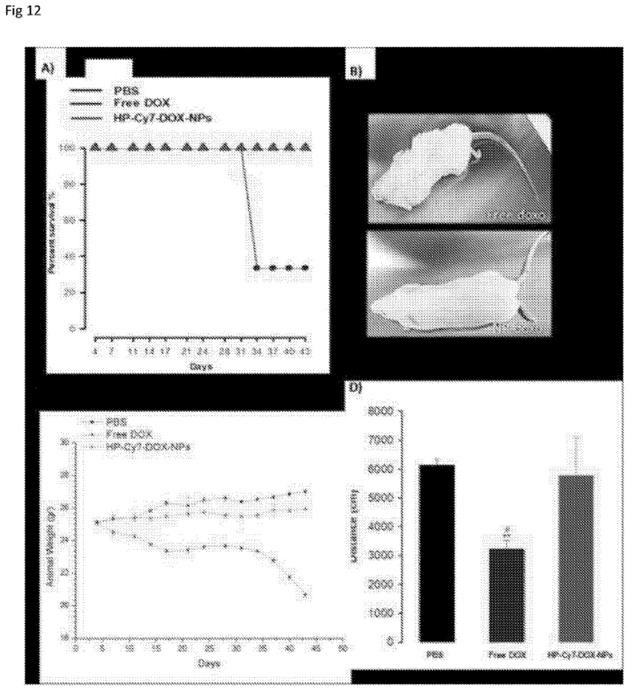

Figure 12. Evaluation of the toxicity of the theranostic nanosystem. A) Kaplan-Meier survival curve. B) Comparative study of the external signs of toxicity in mice treated with free DOX or treated with therapeutic NPs. C) Analysis of the variation in weight during treatment. Each dot and vertical line represents mean ± SEM (n = 5 per group). D) Activity analysis according to the movement capacity (24 hours) of treated mice versus untreated control mice. Statistically significant differences between the control (PBS) and groups treated with free DOX **P≤0.01; and HP-Cy7-DOX-NPs vs. free DOX #P≤0.05 (two-way repeated measures ANOVA followed by the Bonferroni test)
Figure 13. Histological analysis with haematoxylin/eosin and DAPI staining of tumours removed after treatment with PBS (A), free DOX (B), and HP-Cy7-DOX-NPs (C).

### DETAILED DESCRIPTION OF THE INVENTION

### Definitions

For the purpose of the present invention, *"functionalisation"* refers to the introduction of organic polymers or molecules on the surface of the nanoparticle. Given that the nanoparticle of the invention is functionalised with three different types of molecules (T, D, and L) with different functions, it is considered trifunctionalised.

For the purpose of the present invention, *"imaging agent (T)"* refers to any agent that can be used to control treatment or diagnostic efficacy. Examples of this imaging agent may be (non-exhaustive list): organic fluorophores such as bodipys and long-lasting fluorescent dyes for optical imaging, contrast agents for magnetic resonance imaging of cancer such as gadolinium derivatives: gadopentetic acid, gadoteric acid of gadodiamine, and gadoteridol; iron derivatives: ammonium iron(III) citrate, iron(III) oxide and ferroxide; and magnesium derivatives: mangafodipir.

For the purpose of the present invention, *"bioactive molecule (D)"* refers to any therapeutic agent which can be used for treating cancer (or other diseases) or any diagnostic agent which can be used for the diagnosis of cancer, depending on the intended use of the nanoparticle of the invention. Examples of the types of cancer which can be treated or diagnosed with the nanoparticle of the invention are (non-exhaustive list): breast cancer, lung cancer, colorectal cancer, melanoma, etc. Examples of therapeutic agents are (non-exhaustive list): doxorubicin, epirubicin, paclitaxel, 5-FU, gemcitabine, cyclophosphamide, eribulin, capecitabine, etc. In this invention, the therapeutic agent of choice is doxorubicin used for the treatment of breast cancer. Furthermore, the nanoparticle of the invention may comprise, at the same time, more than one bioactive molecule (D) that would be designed using chemical conjugation methods, giving rise to a versatile and highly specific diagnostic platform, by means of the application of orthogonal strategies which allow multifunctionalisation.

For the purpose of the present invention, *"ligand* (*L*)*"* refers to any molecule, particularly any tumour-specific peptide targeting tumour to be treated or diagnosed. The ligand or peptide of reference can detect tumour *in vivo* and release anti-cancer agents specifically in the tumour. Examples of ligands may be (non-exhaustive list): peptide ligands targeting other biological targets (for example, TEM5 and TEM8, DDL4, etc.) as well as monoclonal antibodies (for example, EFGR, HER2, etc.).

### Method for producing the nanoparticles

Therefore, the first embodiment of the present invention relates to a method (method of the invention) for producing functionalised polystyrene nanoparticles (NP), preferably functionalised amino polystyrene NPs that can be bifunctionalised, comprising the following steps:
a) introducing the NPs in a suitable medium, preferably dimethylformamide (DMF), in which a PEG spacer (or any other suitable spacer) protected, preferably by Fmoc (fluorenylmethoxycarbonyl), preferably Fmoc-4,7,10-trioxa-1,13-tridecanediamine succinamic acid (Fmoc-PEG-OH), is either dissolved and activated in the medium or activated before being dissolved in the medium, for a period of time sufficient for coupling the PEG spacer protected with Fmoc, preferably Fmoc-PEG-OH, to the amino nanoparticles;
b) optionally deprotecting the Fmoc group of the NPs of step a) and then adding one or more PEG spacers protected with Fmoc, preferably Fmoc-4,7,10-trioxa-1,13-tridecanediamine succinamic acid (Fmoc-PEG-OH), in the same manner as described in step a);
c) deprotecting the Fmoc group of the NPs of step a) or b) and then adding one or more amino acids or the analogues thereof, preferably one or more lysine having the N-α-amino and N-ε groups thereof protected by orthogonal protecting groups such as Dde and Fmoc, preferably Fmoc-Lys (Dde); and
d) optionally deprotecting the Fmoc group of the NPs of step c) and then adding one or more PEG spacers protected with Fmoc, preferably Fmoc-4,7,10-trioxa-1,13-tridecanediamine succinamic acid (Fmoc-PEG-OH), in the same manner as described in step a).

In the first embodiment of the present invention, the nanoparticles are bifunctionalised by deprotecting the Fmoc and Dde groups and bonding two chemical groups, used for the bifunctionalisation of the NPs, respectively, to the Dde protecting group-bonded lysine side chain amino group before the deprotection step and to the Fmoc protecting group-bonded amino group before the deprotection step.

In the first embodiment of the present invention, the Fmoc group of the NPs of step d) or c) is deprotected and one or more amino acids or analogues orthogonally protected with Dde and Fmoc, preferably Fmoc-lys- (Dde), is then added.

In the first embodiment of the present invention, the nanoparticles are trifunctionalised by deprotecting the Fmoc and Dde groups and bonding three chemical groups, used for the trifunctionalisation of the NPs, at least two amino groups of the lysine side chain, respectively, bonded to the Dde groups before the deprotection step and to the Fmoc group-bonded amino before the deprotection step.

In the first embodiment of the present invention, the trifunctionalisation of the NPs is performed by bonding to the NPs a chemical group comprising two PEG spacers that are orthogonally protected, preferably with Fmoc, preferably Fmoc-4,7,10-trioxa-1,13-tridecanediamine succinamic acid (Fmoc-PEG-OH), said spacers each having two units and two amino acids or analogues having the N-α-amino and N-ε groups thereof protected by orthogonal protecting groups such as Dde and Fmoc, preferably Fmoc-Lys (Dde).

In the first embodiment of the present invention, a first PEG spacer having two units is coupled directly to the NP; a first amino acid or analogue, preferably orthogonally protected lysine, is coupled directly to the amino group of the first PEG spacer; the second PEG spacer is coupled directly to the alpha-amino group of the first lysine group, and the second lysine group is coupled directly to the amino group of the second PEG spacer.

In the first embodiment of the present invention, the nanoparticles are trifunctionalised by deprotecting the Fmoc and Dde groups and coupling three chemical groups, respectively, at least two amino groups of the lysine side chain, respectively, bonded to the Dde groups before the deprotection step and to the Fmoc group-bonded amino before the deprotection step.

In the first embodiment of the present invention, the NPs are characterised by being cross-linked by means of divinylbenzene.

In the first embodiment of the present invention, the nanoparticle is trifunctionalised with (a) at least one imaging agent (T), at least one bioactive molecule (D), and at least one ligand (L).

In the first embodiment of the present invention, the imaging agent (T) is a fluorophore, preferably a far-red cyanine derivative (Cy7).

In the first embodiment of the present invention, the bioactive molecule (D) is a therapeutic agent, a diagnostic agent, or a drug, preferably doxorubicin.

In the first embodiment of the present invention, the ligand (L) is a tumour-specific peptide or peptidomimetic, preferably the homing peptide RGD.

### Nanoparticles of the invention

The second embodiment of the present invention relates to a polystyrene or amino polystyrene nanoparticle (nanoparticle of the invention) trifunctionalised preferably with (a) at least one imaging agent (T), at least one bioactive molecule (D), and at least one ligand (L), wherein said nanoparticle is bonded to a chemical group comprising two PEG spacers protected with Fmoc, preferably Fmoc-4,7,10-trioxa-1,13-tridecanediamine succinamic acid (Fmoc-PEG-OH), said spacers each having two units and two amino acids or analogues, preferably a lysine having the N-α-amino and N-E groups thereof protected by orthogonal protecting groups such as Dde and Fmoc, preferably Fmoc-Lys (Dde).

In the second preferred embodiment of the nanoparticle of the invention, a first PEG spacer having two units is bonded directly to the NPs; a first lysine group is bonded directly to the amino group of the first PEG spacer; the second PEG spacer is coupled directly to the alpha-amino group of the first lysine group, and the second lysine group is coupled directly to the amino group of the second PEG spacer.

In the second preferred embodiment of the nanoparticle of the invention, the size range of the nanoparticle is from 100 nm to 2000 nm. Preferably, the nanoparticle of the invention has a size of about 200 nm.

In the second preferred embodiment of the nanoparticle, the ligand (L) is a tumour-specific peptide or peptidomimetic, preferably the homing peptide RGD.

In the second preferred embodiment of the nanoparticle of the invention, the bioactive molecule (D) is a therapeutic agent, a diagnostic agent, or a drug, preferably doxorubicin.

In the second preferred embodiment of the nanoparticle, the imaging agent (T) is a fluorophore, preferably a far-red cyanine derivative (Cy7).

### Nanodevice of the invention

The third embodiment of the present invention relates to a nanodevice, particularly a diagnostic nanodevice, comprising any of the nanoparticles of the invention described in the second embodiment of the invention or a nanoparticle obtained by means of the method of the invention.

### Uses of the nanoparticles of the invention

The fourth embodiment of the present invention relates to the nanoparticle, the nanodevice, or the nanoparticle obtained by means of the method of the invention, for use thereof in the treatment of cancer, the diagnosis of cancer, or in the control of the treatment of cancer.

### Pharmaceutical composition

The fifth embodiment of the present invention relates to a pharmaceutical composition comprising the nanoparticle or the nanoparticle obtained by means of the method of the invention.

### EXAMPLES

### Example 1. Polymer nanoparticle (NP-1).

The nanoparticle of the invention can be made of the following materials (non-exhaustive list): polystyrene, 4-aminostyrene, styrene, amino polystyrene, polylactic acid (PLA), poly(lactic-co-glycolic acid) (PLGA), poly(N-vinylpyrrolidone) (PVP), polyethylene glycol, polycaprolactone, polyacrylic acid (PAA), poly(methyl methacrylate), polyacrylamide, chitosan, gelatin, or sodium alginate, among other polymers. In a particular embodiment, the nanoparticle of the invention is made of polystyrene or 4-aminostyrene.

Furthermore, the nanoparticle of the invention is cross-linked with divinylbenzene (DVB), 1,4-bis(4-vinylphenoxy)butane, bis(2-methacryloyl)oxyethyl disulfide, azobisisobutyronitrile (AIBN), 2,2'-azobis(2-methylpropionamidine) dihydrochloride, or benzoyl peroxide (BPO). Preferably, the nanoparticle of the invention is cross-linked with divinylbenzene.

In another particular embodiment, the nanoparticle of the invention is functionalised with amino groups, carboxyl groups (particularly, carboxylic acid), maleimide, azide, alkyne, tetrazine, thiol, cyclooctyne, alcohol groups. Preferably, the nanoparticle of the invention is functionalised with amino groups (amino-functionalised).

In an even more preferred embodiment, the nanoparticle of the invention is polystyrene, cross-linked with divinylbenzene, and functionalised with amino groups.

### Example 2. Protocols for conjugating bioactive loads to the nanoparticles

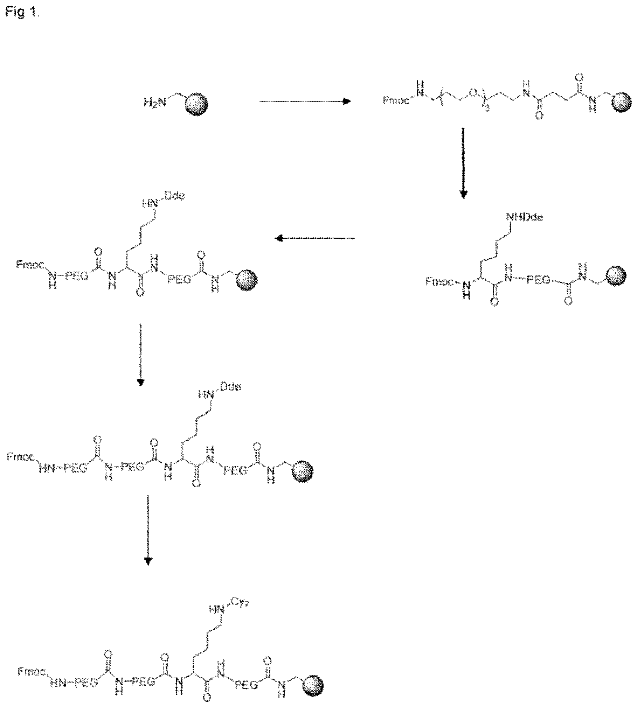

**Example 2.1:** Aminomethyl polystyrene nanoparticles (NP-1) were functionalised as described below and summarised in Figure 1 following a standard solid-phase Fmoc protocol, using oxyma and DIC as coupling reagents. The NPs were functionalised with the polyethylene glycol (PEG) spacer as follows: the NPs were washed and resuspended in dimethylformamide (DMF). At the same time, Fmoc-4,7,10-trioxa-1,13-tridecanediamine succinamic acid (Fmoc-PEG-OH) (50 eq) was dissolved in DMF and activated with oxyma and N,N'-diisopropylcarbodiimide (DIC) (50 eq). This solution was added to the NPs and stirred for two hours at 60°C. The deprotection of the Fmoc (fluorenylmethoxycarbonyl) group of the NPs was then carried out by treating with 20% piperidine/DMF (3 x 20 minutes). For the bifunctionalisation of NPs, Fmoc-Lys (Dde)-OH (50 eq), previously activated with oxyma and DIC (50 eq) in DMF, was added for two hours at 60°C. Two PEG spacers were then added in the same manner as described above. The NPs-1 (Cy7-NPs) were labelled with fluorophore Cy7 (cyanine 7) used for labelling NPs to obtain fluorescence images and the deprotection of the Dde protecting group was carried out with a solution of hydroxylamine.HCl (0.4 mmol) and imidazol (0.3 mmol) in NMP (1 ml) for 1 hour, after which fluorophore Cy7 (1 eq) was added to the NPs in the presence of DIPEA (2 eq) in DMF and stirred for 15 hours at 25°C.

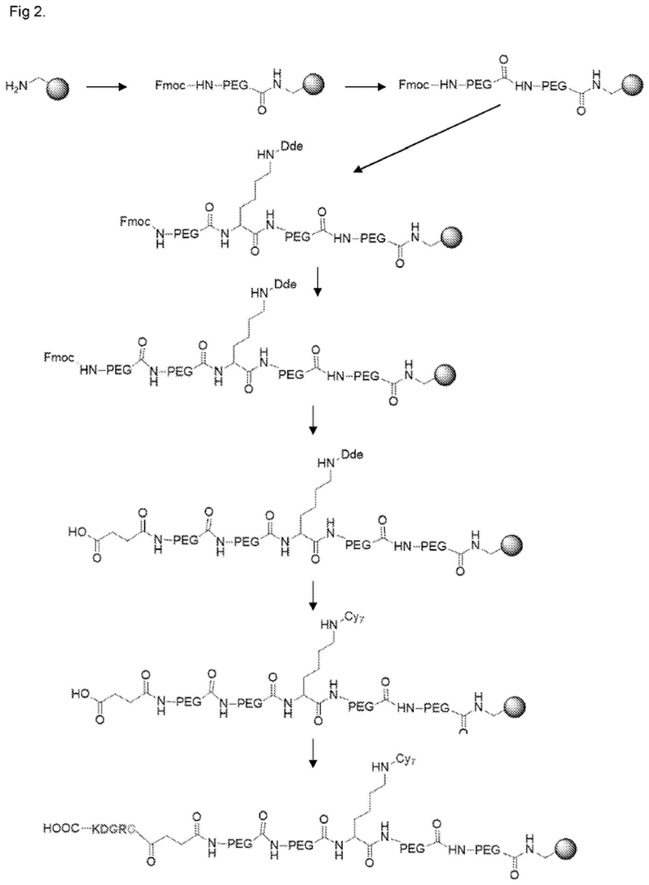

**Example 2.2:** As shown in Figure 2, the NPs-2 (HP-Cy7-NPs) were functionalised with the homing peptide (HP) RGD. For this, after the deprotection of the Fmoc group, succinic anhydride (50 eq) and DIPEA (50 eq) were added to the NPs and stirred for two hours at 60°C. The Dde protecting group was then deprotected and the NPs were labelled with Cy7 as described above. Lastly, the NPs were activated with oxyma and DIC (50 eq) for 4 hours at 25°C, after which HP (7 eq) in DMF with DIPEA was added and stirred for 15 hours at 25°C.

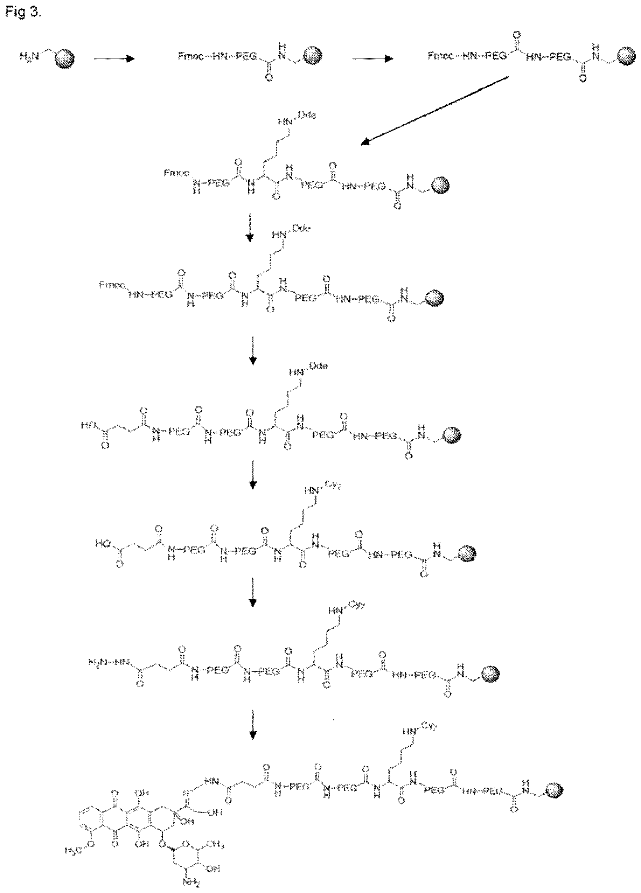

**Example 2.3:** As shown in Figure 3, the NPs-3 (Dox-Cy7-NPs) were functionalised with doxorubicin (Dox). For this, the deprotection of the Fmoc group was performed and succinic anhydride (50 eq) was conjugated with DIPEA (25 eq) and stirred for 2 h at 60°C. The Dde group was then deprotected and the NPs were labelled with Cy7 as described above. The NPs were activated with oxyma and DIC (50 eq) for 4 hours at 25°C, and a 55% v/v (50 eq) solution of hydrazine in DMF was added and left under stirring for 15 hours at 25°C. The NPs were subsequently washed with PBS at pH 6. Doxorubicin (1.6 mg, 1 eq) in PBS at pH 6 was dissolved and added to the NPs and the resulting mixture was left under stirring for 15 hours at 50°C. Lastly, the NPs were washed with PBS at pH 7.4.

### Example 3. Trifunctionalisation of nanoparticles

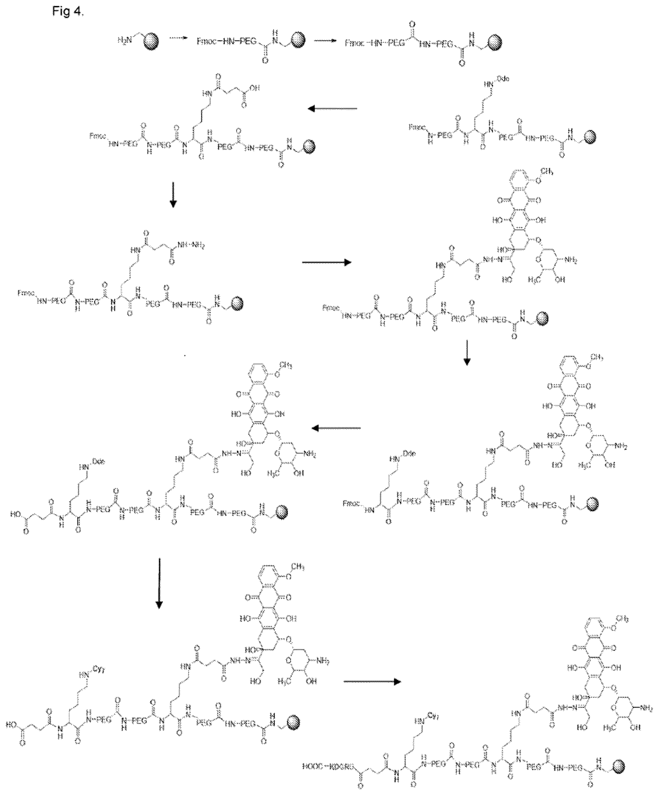

For the trifunctionalisation of NPs-4 (Dox-HP-Cy7-NPs) (see Figure 4), the nanoparticles are doubly PEGylated with two units of PEG spacer and bifunctionalised using a lysine orthogonally protected with Dde and Fmoc. The nanoparticles were then functionalised with a carboxyl group (using succinic anhydride) and treated with hydrazine before conjugation with the drug through a hydrazone linkage (pH-sensitive linker). In the next step, a second coupling with Fmoc-Lys (Dde)-OH was performed for the trifunctionalisation of the nanoparticle. Carboxyl-functionalisation of the NPs was carried out, followed by the fluorescent labelling thereof using a red cyanine derivative (Cy7). This labelling will allow tracking the nanoparticles by means of using fluorescence-based techniques. Lastly, the homing peptide functionalised with an aminooxy group will allow site-specific chemoselective bonding to carboxyl-functionalised nanoparticles. Briefly, the carboxyl-functionalised nanoparticles were preactivated with oxyma and DIC (25 eq) in DMF for 2 hours at 25°C before adding the homing peptide functionalised with the aminooxy group (20 equivalents) and mixed at 25°C for 18 hours for producing the desired nanoconjugates. The deprotection of the Dde group was then carried out and succinic anhydride (50 eq) and DIPEA (50 eq) were added to the NPs and the solution was stirred for two hours at 60°C. The NPs were activated with oxyma and DIC (50 eq) for 4 hours at 25°C, after which a 55% v/v (50 eq) solution of hydrazine in DMF was added and left under stirring for 12 hours at 25°C. The NPs were subsequently washed with PBS at pH 6. Doxorubicin in PBS at pH 6 was added to the NPs and the resulting mixture was left under stirring for 15 h at 50°C. The deprotection of the Fmoc group was then carried out and a second Fmoc-Lys-OH was added, the Fmoc group was removed and succinic anhydride conjugated. The Dde group was deprotected again and the NPs were labelled with Cy7 as described above. Lastly, the NPs were activated with oxyma and DIC (50 eq) for 4 h at 25°C, after which HP (7 eq) in DMF with DIPEA was added for 15 h at 25°C.

### Example 4. Evaluation of breast cancer cell NP uptake efficacy

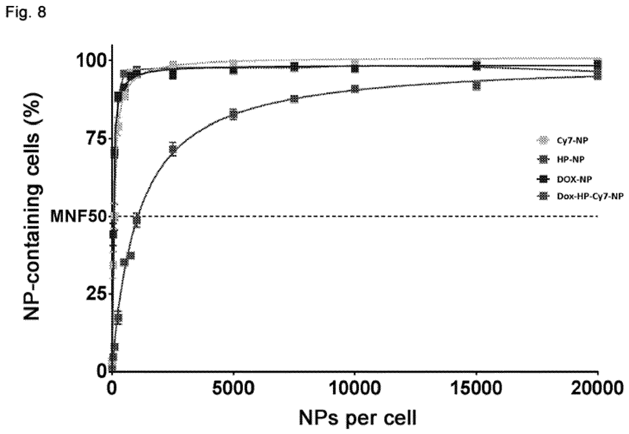

The MDA-MB-231 breast cancer cell line was used as a cell model to evaluate the uptake efficacy of these nanoparticles. Several assays were carried out to determine the number of nanoparticles required to achieve 50% of NP cellular uptake in the cell population (MNF₅₀). A range of nanoparticle concentrations from 50 to 10,000 NPs/cells was evaluated for 24 hours. Figure 8 shows the results obtained by means of flow cytometry. The uptake of trifunctionalised nanoparticles, Cy7-HP-Dox-NPs, is highly efficient with an MNF₅₀ value of 1700 NPs/cell. The uptake was compared with the control nanoparticles: Cy7-NPs, Cy7-Dox-NPs, Cy7-HP-NPs. A time study was conducted to evaluate the effect of incubation time on nanoparticle uptake. Time is an important factor for nanofection, i.e., the longer the incubation time, the fewer NPs are required. The cellular uptake of Dox-Cy7-NPs is compared with HP-Dox-Cy7-NPs for a period of 72 hours in different time intervals (3, 6, 24, 48, and 72 hours), with a fixed number of NPs (5,000 NPs/cell). As can be seen in the confocal microscopy images obtained 3 hours after incubation, the nanofection of the cells has already been achieved, with there being a clear difference between both types of NPs, where Dox-Cy7-NPs reach a nanofection of 75% compared with HP-Dox-Cy7-NPs of 15%. Meanwhile, both NPs reach a nanofection of 100% after 24 hours. Based on these results, it can be concluded that the breast cancer cells efficiently uptake the trifunctionalised NPs obtained after 24 hours of incubation. Curiously, the presence of the homing peptide on the surface of the trifunctionalised NPs reduces the amount of NPs taken up within a short time (3 h) when compared with NPs without the targeting ligand. This result reinforces the fact that the uptake of these trifunctionalised NPs is selective.

### Example 5. Therapeutic efficiency

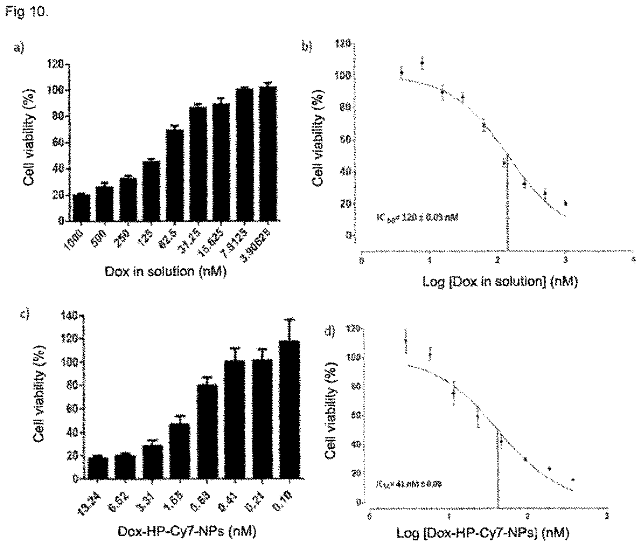

Cell viability studies were conducted to evaluate the effects of the drug (doxorubicin) in solution and conjugated to the NPs on triple-negative MDA-MB-231 breast cancer cells. The cytotoxicity of Dox was studied using resazurin, a quantitative colorimetric method to determine cell viability. For cell viability evaluation, the cells were incubated with different concentrations of doxorubicin in solution and with a different number of HP-Dox-Cy7-NPs, as shown in Figure 10, for 120 hours (5 days). The capacity of doxorubicin to inhibit cell growth by 50%, IC₅₀, of the population with respect to untreated cells was then determined by means of the resazurin colorimetric assay. The IC₅₀ of doxorubicin in solution is 1.67 nM, whereas the IC₅₀ of NP-conjugated doxorubicin is 0.1 nM, which corresponds with 2,200 NPs/cell. This demonstrates the efficacy of NP-bonded doxorubicin as it maintains the IC₅₀ value with respect to Dox in solution, making HP-Dox-Cy7-NP a highly effective proliferative agent. Surprisingly, the dose of anti-tumour medicinal product (Dox) to achieve IC₅₀ is much lower than the dose required in the solution.

### Example 6. In vivo evaluation. Breast cancer model/MBA-MD-231/xenographic model

All the *in vivo* experiments were performed in NOD scid gamma mice (NSG, NOD.Cg-Prkdcscid II2rgtm1Wjl/SzJ). The animal wellbeing and experimental methods were carried out according to institutional guidelines (Research Ethics Committee of University of Granada, Spain) and international guidelines (European Community Council Directive 86/609). All the animals (n = 5 per group) were kept in a micro-ventilated cage system with a 12-h light/darkness cycle, and handled in a laminar flow cabinet to maintain the specific pathogen-free conditions. To establish orthotopic xenograft tumours, female mice six to eight weeks of age were used. The mice were anaesthetised by means of the inhalation of isoflurane and tumours were generated in the right breast by means of subcutaneous injections of 5x10⁵ cells/mouse mixed with Corning® Matrigel® Matrix and with 26-gauge needles. The intravenous administration of PBS (control), free DOX, and NPs in the tail vein of the mice was performed every three days for a period of 43 days, as was the fluorescence evaluation by means of IVIS® (Perkin Elmer). Tumour growth was evaluated twice a week with a digital calibrator and tumour volume was calculated by means of the formula V = (length) 2 x width x π/6. The weight of the mice was also measured every three days. The endpoint of the experiment was day 44. The mice were left without treatment for one month to eliminate NPs that did not bond specifically to the tumour tissue. During that month, the fluorescence of the mice was analyzed in IVIS® once a week. Lastly, the mice were sacrificed by cervical dislocation and the tumours were extirpated, photographed, and analyzed by means of IVIS®.

Comparative efficacy studies were carried out to investigate the *in vivo* therapeutic efficacy of these therapeutic NPs (HP-Cy7-DOX-NPs). An orthotopic xenotransplantation of the triple-negative human MDA-MB-231 breast cancer cell line was performed in immunocompromised female NSG mice. The mice (5 per group) were divided into 3 groups and treated according to the protocol approved by the Institutional Animal Care and Use Committee (IACUC) of the University of Granada and the Regional Government of Andalusia according to the European Directive on the protection of animals used for scientific purposes (2010/63/EU) and Spanish law (Royal Decree 53/2013). Treatment with PBS (control), free DOX, and NPs (HP-Cy7-DOX-NPs) was administered for 43 days, with the time when the tumour reached a size of 100 mm³ being considered time zero. Periodic intravenous administrations to each group of mice were performed every 3 days. Mice treated with DOX in solution showed a significant inhibition of tumour growth after 3 weeks (Figure 11). This therapeutic effect was evident for the therapeutic NPs after 28 days, where a decrease in the tumour volume that was reached on day 43 was observed, as it is similar to that of mice treated with free DOX (Figures 11A and 11B). These results suggest that HP-Cy7-DOX-NPs exhibit a clear therapeutic effect similar to that of free DOX. Surprisingly, fluorescence intensity analysis by means of IVIS showed that the trifunctionalised NPs (HP-Cy7-DOX-NPs) accumulated selectively in the mass of the breast tumour and not in the rest of the organs (Figure 11C).

Histological analysis of orthotopic xenograft tumour tissues further confirmed the therapeutic efficacy of HP-Cy7-DOX-NPs. The xenograft tumours of the control group consisted of very compact, proliferative tumour cells (Figure 12). However, in the xenograft tumours of the treatment groups, cellularity decreased significantly, with typical apoptotic characteristics, such as small nuclear fragments surrounded by a clear cytoplasmic border, most often observed in tumours treated with NPs than in those treated with DOX (Figures 11B and 11C).

These *in vivo* findings demonstrate the efficient targeted administration and the improved therapeutic activity of these therapeutic NPs in Nrp-1 overexpressing triple negative breast cancer tumours. It has also been previously reported that nanomedicine functionalised with CRGD peptides offered good targeting capacity for MDA-MB-231 cells *in vitro* and *in vivo.*

### Example 7. Toxicity assays

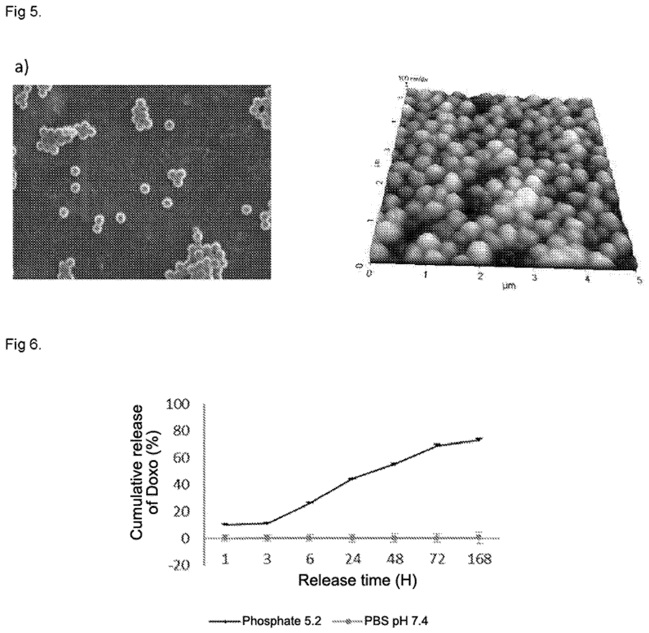

In another assay, a series of experiments were carried out to control the *in vivo* toxicity of the treatment. First, a comparative analysis of the survival of mice treated with the therapeutic nanoparticles (HP-Cy7-DOX-NPs) with respect to mice treated with DOX in solution and untreated control mice (PBS) was carried out during the treatment period (43 days). The percentage of survival (Figure 12) during the treatment period was 100% both for mice treated with NPs and for controls. However, treatment with DOX caused a high impact on survival. After 30 days of treatment, there was a drastic decrease in the percentage of survival (30%). This effect could be attributed to the inherent systemic toxicity of this anti-tumour drug and to the serious side effects of this conventional chemotherapy treatment. In fact, an analysis of the physical appearance of the treated mice corroborates this result. Figure 6B shows a representative image of a mouse treated with free DOX, in which obvious external signs of toxicity are observed compared with a mouse from the group treated with theranostic nanoparticles (HP-Cy7-DOX-NPs).

The main external side effects observed in mice treated with free DOX were weight loss, goose bumps, behaviour disorders, and damaged tail, among others. The most surprising side effect was the high cutaneous toxicity caused in the tail of mice treated with DOX (Figure 12B, top image). Furthermore, a significant weight reduction was observed in mice treated with free DOX compared with the weight of untreated control mice or mice treated with theranostic NPs (Figure 12C). Furthermore, in order to observe the toxic effect of the treatment at the central nervous system level, a standardised protocol for testing movement was carried out on day 30 in a cage with a camera prepared for this purpose. This camera analyzes the distance the mouse travels in 24 hours. Treatment with HP-Cy7-DOX-NPs did not affect motor activity, being comparable to that of untreated mice; however, free DOX caused a significant decrease in mouse mobility (Figure 12C). In metastatic breast cancer, treatment with DOX exhibits several adverse effects including cardiotoxicity, haematological toxicity, and cutaneous toxicity due to the lack of selectivity, which subsequently causes therapeutic failure.

The assays described in Examples 6 and 7 show that these therapeutic nanoparticles (HP-Cy7-DOX-NPs) have a high selectivity with respect to triple negative breast tumours due to conjugation with the RGD peptide and have a therapeutic effect similar to free DOX after 43 days of treatment, but with a notable advantage, these NPs did not produce detectable side effects.

### Example 8. Competitiveness study

Although the data obtained up until now showed a specific uptake of theranostic nanoparticles (HP-Cy7-DOX-NPs) as a result of them targeting the Nrp-1 receptor, a more in-depth study was conducted to verify these results. Competitive binding experiments with the CRGDK peptide in solution were performed in the culture medium. The cells were pre-incubated with the CRGDK peptide for 6 hours (EGFR +) and then treated with HP-Cy7-DOX-NPs under the same conditions as in the preceding assay. Under the pre-incubation conditions of the CRGDK peptide, cell binding sites were effectively blocked, preventing epitope recognition from nanodevices and thereby preventing the uptake of HP-Cy7-DOX-NPs. The cellular uptake of HP-Cy7-DOX-NPs with the free pre-incubated CRGDK peptide was significantly reduced. Furthermore, the degree of uptake of CRGDK peptide-free nanoparticles remained constant despite the pre-incubation with the CRGDK peptide in solution (Figure 13). This data also supports the HP-Cy7-DOX-NP receptor-mediated and -specific binding mechanism and improvement in the active targeting of cancer cells overexpressing Nrp-1.

### Example 9. Detailed description of the conjugation protocols for multifunctionalisation

1. The NPs of the invention were synthesised by means of dispersion polymerization.
2. To carry out the step of conjugating the amino groups of NPs-1 and the acids of fluorophore Cy7 (cyanine 7), the nanoparticles were previously conditioned by means of washing three times with dimethylformamide (DMF), through suspension-centrifugation cycles (13,400 rpm, 3 minutes). Then, 50 equivalents of Fmoc-4,7,10-trioxa-1,13-tridecanediamine succinamic acid (Fmoc-PEG-OH) were dissolved in 1 mL of DMF together with 50 equivalents of oxyma and 50 equivalents of N,N'-diisopropylcarbodiimide (DIC). The mixture was stirred at room temperature for 10 minutes. After that time, the solution was added to the dry nanoparticles, suspended, and the suspension was left under stirring at 60°C and 1,400 rpm for 2 hours.
3. After 2 hours, the nanoparticles were washed with three suspension-centrifugation cycles (13,400 rpm, 3 minutes) to obtain the Fmoc-PEGylated nanoparticles. The deprotection of the Fmoc (fluorenylmethoxycarbonyl) protecting group was carried out by means of a treatment consisting of three 20-minute washings with 1 mL of 20% piperidine in DMF. After treatment with piperidine, three washings with 1 mL of DMF (13400 rpm, 3 minutes) were performed by means of suspension-centrifugation cycles. Once the Fmoc group has been removed by means of treatment with 20% piperidine in DMF, the NPs were PEGylated again following the method described in the preceding paragraph. After the three NP washing cycles (13400 rpm, 3 minutes), the deprotection of the Fmoc group was performed by means of treatment with 20% piperidine in DMF and the NPs were washed again. Then, 50 equivalents of Fmoc-Lys-Dde(OH) were dissolved in 1 mL of DMF together with 50 equivalents of oxyma and 50 equivalents of DIC. The mixture was stirred at room temperature for 10 minutes. After that time, the solution was added to the dry NPs, suspended, and the suspension was left under stirring at 60°C and 1,400 rpm for 2 hours.
4. The NPs were washed again with three suspension-centrifugation cycles (13,400 rpm, 3 minutes), the Fmoc protecting group was removed by means of treatment with 20% piperidine in DMF, the NPs were washed with three suspension-centrifugation cycles (13,400 rpm, 3 minutes), and a solution of 50 equivalents of Fmoc-PEG-OH with 50 equivalents of oxyma and 50 equivalents of DIC, previously stirred at room temperature for 10 minutes, was added to the NPs. Once added, the NPs were resuspended in said solution and left under stirring at 60°C and 1,400 rpm for 2 hours.
5. The entire process described in paragraph 4 was repeated again.
6. The NPs were washed with three suspension-centrifugation cycles (13,400 rpm, 3 minutes). The deprotection of the Dde group present in the Fmoc-Lys-Dde(OH) was performed by means of a treatment with a 0.4 mmol solution of hydroxylamine.HCl and 0.3 mmol of imidazol dissolved in 1 ml of N-methyl-pyrrolidone (NMP) for 1 hour at room temperature.
7. Next, the NPs were washed with three suspension-centrifugation cycles (13,400 rpm, 3 minutes), the Fmoc protecting group was removed by means of treatment with 20% piperidine in DMF, the NPs were washed with three suspension-centrifugation cycles (13,400 rpm, 3 minutes), 0.1 eq of Cy7 was simultaneously dissolved together with 0.1 eq of N,N-diisopropylethylamine (DIPEA) in DMF, and this solution was added to the dry NPs, suspended, and the suspension was left under stirring for 15 hours at room temperature. After conjugation, the NPs were washed with three suspension-centrifugation cycles (13,400 rpm, 3 minutes) and resuspended in 1 ml of Milli-Q water.
8. To conjugate the homing peptide RGD to NPs-2, conjugations described in paragraphs 1-5 were performed. Next, the NPs were washed with three suspension-centrifugation cycles (13,400 rpm, 3 minutes) and the deprotection of the Fmoc group was carried out by means of treatment with 20% piperidine in DMF, the NPs were washed with three suspension-centrifugation cycles (13,400 rpm, 3 minutes), and a solution of 50 equivalents of succinic anhydride and 50 equivalents of DIPEA, previously stirred at room temperature for 2 minutes, was added to the dry NPs. Once added, the NPs were resuspended in said solution and left under stirring at 60°C and 1,400 rpm.
9. The deprotection of the Dde group and the conjugation of fluorophore Cy7 were carried out according to the methods described in paragraphs 6-7.
10. Lastly, the NPs were washed with three suspension-centrifugation cycles (13,400 rpm, 3 minutes) and the carboxyl groups of the NPs were activated by means of adding a solution of 50 equivalents of oxyma and 50 equivalents of DIC in 1 mL of DMF, the NPs were suspended and left under stirring for 4 hours at room temperature. After 4 hours, the NPs were centrifuged and a solution of 7 equivalents of the homing peptide RGD and 0.1 equivalent of DIPEA in 1 ml of DMF was added. It was left under stirring for 15 hours at room temperature. After conjugation, the NPs were washed with three suspension-centrifugation cycles (13,400 rpm, 3 minutes) and resuspended in 1 ml of Milli-Q water.
11. The conjugation of doxorubicin to NPs-3 was carried out following the conjugations described in paragraphs 1-5 and 8-9.
12. Next, the NPs were washed with three suspension-centrifugation cycles (13,400 rpm, 3 minutes) and activated by means of adding a solution of 50 equivalents of oxyma and 50 equivalents of DIC in 1 mL of DMF; the NPs were suspended and left under stirring for 4 hours at room temperature. After 4 hours, the NPs were centrifuged and a solution with 50 equivalents of 55% v/v hydrazine in 1 mL of DMF was added and left under stirring for 15 hours at 25°C. The NPs were then washed and conditioned in 1 mL of PBS pH 6 by means of 3 suspension-centrifugation cycles (13,400 rpm, 3 minutes). Lastly, 1 equivalent of doxorubicin was dissolved in 1 mL of PBS at pH 6 and added to the NPs, and the resulting mixture was left under stirring for 15 hours at 50°C. Finally, the NPs were washed with three suspension-centrifugation cycles (13,400 rpm, 3 minutes) and resuspended in 1 mL of PBS at pH 7.4.
13. The trifunctionalisation of NPs-4 was performed following the conjugations described in paragraphs 1-6. The NPs were then washed with three suspension-centrifugation cycles (13,400 rpm, 3 minutes) and succinic anhydride was conjugated according to paragraph 8.
14. The conjugation of doxorubicin was carried out as described in paragraph 12, but the NPs were ultimately resuspended in 1 mL of DMF.
15. The NPs were washed with three NP washing cycles (13400 rpm, 3 minutes), the deprotection of the Fmoc group was performed by means of treatment with 20% piperidine in DMF, and the NPs were washed again. Next, Fmoc-Lys-Dde(OH) was bonded as described in paragraph 3. The deprotection of the Fmoc group was performed again by means of treatment with 20% piperidine in DMF and the NPs were washed again and succinic anhydride was conjugated as explained in paragraph 8.
16. The deprotection of the Dde group and the conjugation of fluorophore Cy7 were carried out according to the methods described in paragraphs 6-7.
17. Lastly, the activation and conjugation of NPs to the homing peptide RGD was performed as described in paragraph 10.

## Claims

1. Method for producing functionalised polystyrene nanoparticles (NPs), preferably functionalised amino polystyrene NPs that can be bifunctionalised, comprising the following steps:
a) introducing the NPs in a suitable medium, preferably dimethylformamide (DMF), in which a PEG spacer (or any other suitable spacer) protected, preferably by Fmoc (fluorenylmethoxycarbonyl), preferably Fmoc-4,7,10-trioxa-1,13 -tridecanediamine succinamic acid (Fmoc-PEG-OH), is either dissolved and activated in the medium or activated before being dissolved in the medium, for a period of time sufficient for coupling the PEG spacer protected with Fmoc, preferably Fmoc-PEG-OH, to the amino nanoparticles;
b) optionally deprotecting the Fmoc group of the NPs of step a) and then adding one or more PEG spacers protected with Fmoc, preferably Fmoc-4,7,10-trioxa-1,13-tridecanediamine succinamic acid (Fmoc-PEG-OH), in the same manner as described in step a);
c) deprotecting the Fmoc group of the NPs of step a) or b) and then adding one or more amino acids or the analogues thereof, preferably one or more lysines having the N-α-amino and N-ε groups thereof protected by orthogonal protecting groups such as Dde and Fmoc, preferably Fmoc-Lys (Dde); and
d) optionally deprotecting the Fmoc group of the NPs of step c) and then adding one or more PEG spacers protected with Fmoc, preferably Fmoc-4,7,10-trioxa-1,13-tridecanediamine succinamic acid (Fmoc-PEG-OH), in the same manner as described in step a).

2. Method for producing bifunctionalised polystyrene nanoparticles (NPs), preferably functionalised amino polystyrene NPs, comprising the steps according to claim 1, wherein the nanoparticles are bifunctionalised by deprotecting the Fmoc and Dde groups and coupling two chemical groups, used for the bifunctionalisation of the NPs, respectively, to the Dde-bonded amino group of the lysine side chain before the deprotection step and to the Fmoc-bonded amino group before the deprotection step.

3. Method for producing polystyrene nanoparticles (NPs), preferably functionalised amino polystyrene NPs, which can be trifunctionalised, comprising the steps according to claim 1 and an additional step comprising:
e deprotecting the Fmoc group of the NPs of step d) or c) and then adding one or more amino acids or analogues orthogonally protected with Dde and Fmoc, preferably Fmoc-lysine (Dde).

4. Method for producing trifunctionalised polystyrene nanoparticles (NPs), comprising the steps according to claim 3, wherein the nanoparticles are trifunctionalised by deprotecting the Fmoc and Dde groups and bonding three chemical groups, used for the trifunctionalisation of the NPs, respectively, at least two amino groups of the lysine side chain, respectively, bonded to the Dde groups before the deprotection step and to the Fmoc group-bonded amino before the deprotection step.

5. Method for producing polystyrene nanoparticles (NPs) or functionalised polystyrene NPs which can be trifunctionalised, wherein the trifunctionalisation of the NPs is performed by bonding to the NPs a chemical group comprising two PEG spacers that are orthogonally protected, preferably with Fmoc, preferably Fmoc-4,7,10-trioxa-1,13-tridecanediamine succinamic acid (Fmoc-PEG-OH), said spacers each having two units and two amino acids or analogues having the N-α-amino and N-ε groups thereof protected by orthogonal protecting groups such as Dde and Fmoc, preferably Fmoc-Lys (Dde).

6. The method according to claim 3, wherein a first spacer having two PEG units is coupled directly to the NPs; a first amino acid or analogue, preferably orthogonally protected lysine, is coupled directly to the amino group of the first PEG spacer; the second PEG spacer is coupled directly to the alpha-amino group of the first lysine group, and the second lysine group is coupled directly to the amino group of the second PEG spacer.

7. Method for producing functionalised polystyrene nanoparticles (NPs) or amino-functionalised nanoparticles, comprising the steps according to any of claims 5 or 6, wherein the nanoparticles are trifunctionalised by deprotecting the Fmoc and Dde groups and bonding three chemical groups, used for the trifunctionalisation of the NPs, respectively, at least two amino groups of the lysine side chain, respectively, bonded to the Dde groups before the deprotection step and to the Fmoc group-bonded amino before the deprotection step.

8. Method according to any of the preceding claims, wherein the NPs are **characterised by** being cross-linked with divinylbenzene.

9. Method according to any of claims 4 or 7, wherein the nanoparticle is trifunctionalised with (a) at least one imaging agent (T), at least one bioactive molecule (D), and at least one ligand (L).

10. Polystyrene or amino polystyrene nanoparticle trifunctionalised preferably with (a) at least one imaging agent (T), at least one bioactive molecule (D), and at least one ligand (L), wherein said nanoparticle is bonded to a chemical group moiety comprising two PEG spacers protected with Fmoc, preferably Fmoc-4,7,10-trioxa-1,13-tridecanediamine succinamic acid (Fmoc-PEG-OH), said spacers each having two units and two amino acids or analogues, preferably a lysine having the N-α-amino and N-ε groups thereof protected by orthogonal protecting groups such as Dde and Fmoc, preferably Fmoc-Lys (Dde).

11. Nanoparticle according to claim 10, wherein a first PEG spacer having two units is coupled directly to the NPs; a first lysine is bonded directly to the amino group of the first PEG spacer; the second PEG spacer is coupled directly to the alpha-amino group of the first lysine group, and the second lysine group is coupled directly to the amino group of the second PEG spacer.

12. Nanoparticle according to any of claims 10 or 11, wherein the size range of the nanoparticle is from 100 nm to 2000 nm, preferably about 200 nm.

13. Nanoparticle according to any of claims 10 to 12 or obtained by means of the method according to claim 9, wherein the bioactive molecule (D) is a therapeutic agent, a diagnostic agent, or a drug, preferably doxorubicin.

14. Nanoparticle according to any of claims 10 to 13 or obtained by means of the method according to claim 9, wherein the ligand (L) is a tumour-specific peptide or peptidomimetic, preferably a homing peptide RGD.

15. Nanoparticle according to any of claims 10 to 14 or obtained by means of the method according to claim 9, wherein the imaging agent (T) is a fluorophore, preferably a far-red cyanine derivative (Cy7).

16. Nanodevice comprising a nanoparticle according to any of claims 10 to 15.

17. Nanoparticle according to any of claims 10 to 15 or nanodevice of claim 16, for use in the treatment of cancer, the diagnosis of cancer, or in the control of the treatment of cancer.

18. Nanoparticle obtained by means of the method according to claims 1 to 9, for use in the treatment of cancer, the diagnosis of cancer, or in the control of the treatment of cancer.

19. Pharmaceutical formulation comprising a nanoparticle obtained by means of the method according to claims 1 to 9.

20. Pharmaceutical formulation comprising the nanoparticle according to any of claims 10 to 15.
